(19) Europäisches Patentamt
European Patent Office
Office européen des brevets

(11) **EP 3 524 323 A1**

(12) **EUROPEAN PATENT APPLICATION**
published in accordance with Art. 153(4) EPC

(43) Date of publication:
**14.08.2019 Bulletin 2019/33**

(21) Application number: **17858417.3**

(22) Date of filing: **04.10.2017**

(51) Int Cl.:
*A61P 17/02* (2006.01)      *A61K 35/60* (2006.01)
*A61K 38/00* (2006.01)      *A61P 31/04* (2006.01)

(86) International application number:
**PCT/JP2017/036060**

(87) International publication number:
**WO 2018/066580 (12.04.2018 Gazette 2018/15)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **05.10.2016 JP 2016197206**

(71) Applicant: **Maruha Nichiro Corporation**
**Koto-ku, Tokyo 135-8608 (JP)**

(72) Inventors:
• **ENARI, Hiroyuki**
**Tsukuba-shi**
**Ibaraki 300-4295 (JP)**

• **IOHARA, Keishi**
**Tsukuba-shi**
**Ibaraki 300-4295 (JP)**
• **KAMATA, Akira**
**Tsukuba-shi**
**Ibaraki 300-4295 (JP)**
• **KOIZUMI, Daisuke**
**Tsukuba-shi**
**Ibaraki 300-4295 (JP)**

(74) Representative: **Bianchetti Bracco Minoja S.r.l.**
**Via Plinio, 63**
**20129 Milano (IT)**

(54) **WOUND HEALING AGENT HAVING ACTIVITY TO PROMOTE ANTIBACTERIAL PROPERTIES AND WOUND HEALING PROPERTIES**

(57)      An object of the present invention is to provide an active ingredient for a wound healing agent that has high biocompatibility and an antimicrobial activity, as well as a wound healing accelerating activity, which can be applied to supply of a wound healing agent comprising a single active ingredient, which can be produced by a simple production process and production apparatus. According to the present invention, at least one compound selected from a protamine and pharmaceutically acceptable salts thereof is used as an active ingredient for antibacterial activity and wound healing acceleration of a wound healing agent.

Figure 1

EP 3 524 323 A1

**Description**

Technical Field

The present invention relates to a wound healing agent comprising a protamine as an active ingredient and having an antimicrobial activity and a wound healing accelerating activity.

Background Art

It is significant, in a wound, to prevent infection and accelerate healing, and hence, a wound healing agent having these efficacies has been proposed for a treatment of a wound.

As a composition effective for preventing infection and usable in a wound treatment, Patent Literature 1 discloses an antimicrobial composition having pH of 4 to 8, which contains an antimicrobial agent and EDTA (ethylenediamine tetraacetic acid), wherein the antimicrobial agent is selected from iodine and a silver ion-containing compound, and wherein EDTA is a di-, tri- or tetra-basic salt. Patent Literature 1 describes that a biofilm formed in a wound can be destroyed by the antimicrobial composition.

Patent Literature 2 discloses a composition for inhibiting or dispersing a microbial biofilm, which contains (a) protamine sulfate and (b) benzalkonium chloride; or (a) metaperiodate and (b) 5-fluorouracil; or (a) metaperiodate and (b) chlorhexidine; or (a) protamine sulfate and (b) a silver nanoparticle; as well as a composition for inhibiting or dispersing a microbial biofilm containing protamine sulfate and bisguanide. Patent Literature 2 discloses application of these compositions for wound care.

As a composition effective for healing acceleration, Patent Literature 3 discloses a wound healing composition containing a carrier and an angiogenesis factor and a growth factor derived from a platelet. Patent Literature 4 discloses a composition for wound healing acceleration consisting of a microsphere, which is produced from a material selected from the group consisting of a polystyrene and a polystyrene derivatized with a surface group selected from the group consisting of an amino group, a carboxyl group and a sulfate group. Patent Literature 4 discloses protamine and protamine salts such as protamine sulfate, etc. as examples of compounds for introducing a surface group to be used to derivatize a polystyrene.

Non Patent Literature 1 suggested that a silver-containing compound having an antimicrobial action is cytotoxic, and reported that use of silver sulfadiazine should be avoided, if possible, in a wound where an epidermal keratinocyte is actively growing.

Citation List

Patent Literature

Patent Literature 1: JP2016-40294A
Patent Literature 2: JP2012-515726A
Patent Literature 3: JPS62-501628A
Patent Literature 4: JP2002-502413A
Non Patent Literature

Non Patent Literature 1: Atiyeh BS, Costagliola M, Hayek SN, Dibo SA: Effect of silver on burn wound infection control and healing: Review of the literature: Burns, 2007; 33: 139 - 148

Summary of Invention

Technical Problem

According to the antimicrobial composition containing the antimicrobial agent and EDTA described in Patent Literature 1, prevention of infection can be expected due to the antimicrobial agent. On the other hand, however, it is concerned that wound healing may be inhibited by cytotoxicity of a silver ion, iodine, etc., and that silver may be accumulated in an organ, etc., in the body. Further, the antimicrobial composition described in Patent Literature 1 is obtained by combining a plurality of active ingredients, and, therefore, its production process may be complicated, in some cases.

According to the composition for inhibiting or dispersing a microbial biofilm described in Patent Literature 2, inhibition effect against a biofilm can be expected. On the other hand, however, such composition has the following problems:

- Since the composition contains a plurality of active ingredients, its production process is complicated.
- Since the antimicrobial agents including a metal such silver nanoparticles, an inorganic material such as metaperiodate, or bisguanide, etc., are combined, it is necessary to consider influence and an adverse reaction, caused in a living body, by the combined use of these components.
- Its dosage and usage may be restricted in some cases.

Regarding Patent Literature 3, the wound healing composition contains the angiogenesis factor and the growth factor carried on the carrier. However, it is difficult to deal with these factors in the preparation process of the composition. As a result, they may be lost from the carrier or deactivated during the preparation process.

Regarding Patent Literature 4, the composition consisting of a microsphere is superior in that Patent Literature 4 suggests an indirect bacteriostatic function against infection of a wound with Pseudomonas species. On the other hand, since a main component material of the microsphere is polystyrene forming a core, a special step or special production apparatus for synthesizing the polystyrene particles having a suitable minute size is required. Further, a special step or treatment apparatus is required to introduce the surface group such as an amino group onto the surface of the polystyrene core.

Regarding the background art techniques described above,
a wound healing agent having an antimicrobial activity and a wound healing accelerating activity is required, which has further high biocompatibility including no cytotoxicity or no accumulation in the body, even when a single active ingredient is contained, and which can be produced by a simple production method or a simple production apparatus.

An object of the present invention is to provide a wound healing agent having a high biocompatibility, as well as an antimicrobial activity and a wound healing accelerating activity, which can be produced by a simple production method or a simple production apparatus, and which is also applicable to provide a wound healing agent containing a single active ingredient.

Solution to Problem

Conventional antimicrobial wound healing agents containing silver have been commercially sold. As the substrate thereof, nonwoven fabric, a hydrocolloid, a foam material, etc., are used. There are agents including silver ionically bound to the substrate molecule, or a silver salt, an organic silver compound, a silver nanocrystal, etc., complexed with the substrates. However, problems including their cytotoxicity have been suggested as described above, and it has been also reported that the use of sulfadiazine silver should be avoided, if possible, in a wound with active growth of an epidermal keratinocyte (Non Patent Literature 1).

Since a healing agent containing a plurality of components as active ingredients is disadvantageous from the viewpoints of production and quality management, as well as cost, the use of a compound as a single active ingredient is industrially effective.

An antimicrobial activity and a healing accelerating property are reverse functions from the viewpoint of cell activity. The antimicrobial activity is a function to reduce the microbial activity, while the healing accelerating activity is a function to enhance the human cell activity. Therefore, it is difficult usually from such problem to reach the single active ingredient use having these conflicting functions. Although a large number of active ingredients has been disclosed in the related art, regarding the above described views, it has been not examined to try to search a compound alone having the antimicrobial activity and the wound healing accelerating activity, based on a concept that there would be a wound healing agent by a single active ingredient having both the antimicrobial activity and the wound healing accelerating activity. Furthermore, since a very large number of active ingredients has been disclosed in the related art literatures, it has been extremely difficult to subject the known active ingredients to animal experiments, etc., one by one.

As a result of the intensive investigation, the present inventors have newly found that, among various antimicrobial substances, a protamine and pharmaceutically acceptable salts thereof have a wound healing effect, and, thus, the present invention was completed. It is known that a protamine has an antimicrobial activity, but its wound healing effect is unknown up to date.

A wound healing agent according to the present invention is characterised in comprising at least one compound selected from a protamine and pharmaceutically acceptable salts thereof as an active ingredient for antimicrobial treatment and wound healing acceleration.

Patients suffering from wound can be treated for preventing infection and accelerating wound healing by a wound healing agent according to the present invention, which comprises at least one compound selected from a protamine and pharmaceutically acceptable salts thereof, as an active ingredient having antibacterial activity and wound healing acceleration.

A method of using at least one compound selected from a protamine and pharmaceutically acceptable salts thereof in production of a wound healing agent of the present invention includes using at least one compound selected from a protamine and pharmaceutically acceptable salts thereof as an active ingredient for antibacterial activity and wound healing acceleration of the wound healing agent.

The present invention further relates to use of at least one compound selected from a protamine and pharmaceutically acceptable salts thereof for antimicrobial treatment and wound healing acceleration.

The present invention further relates to a treatment method for treating a patient for antimicrobial treatment and wound healing acceleration of a wound, which comprises applying, to a wound, a wound healing agent, which comprises at least one compound selected from a protamine and pharmaceutically acceptable salts thereof, as an active ingredient for treatment of a wound.

Effects of Invention

According to a wound healing agent comprising at least one compound selected from a protamine and pharmaceutically acceptable salts thereof as an active ingredient for wound healing acceleration, an antimicrobial effect and a wound healing accelerating effect for a wound can be obtained. At least one compound selected from a protamine and pharmaceutically acceptable salts thereof can be combined in the wound healing agent as a single active ingredient. Such combination can provide an antimicrobial effect and a wound healing accelerating effect. Therefore, the above compounds are usable for wound healing as an effective wound healing agent in an industrial aspect, based on that they have high biocompatibility and that addition of a single active ingredient may be allowed in production of the wound healing agent.

Brief Description of Drawing

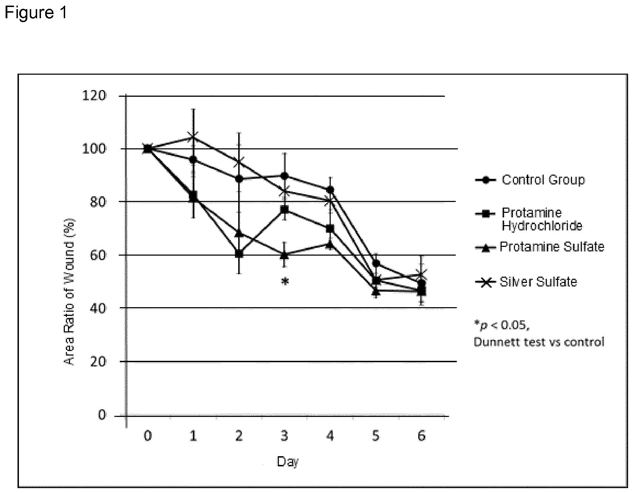

[Figure 1] Figure 1 is a graph showing the results of a healing test in the form of average value ± standard error. It can be confirmed, based on the results illustrated in Figure 1, that a protamine hydrochloride group and a protamine sulfate group exhibit a wound healing accelerating effect, in comparison with a control group (no application) and a silver sulfate group.

Description of Embodiments

A protamine is known to have an antimicrobial activity, and is a highly safe material conventionally used as a food preservative, etc. On the other hand, as a result of studies by the present inventors, it was newly found that a protamine has a wound healing accelerating effect in addition to the antimicrobial activity, and that a wound healing agent having an antimicrobial activity can be provided by using a protamine component as a single active ingredient.

It is considerable that a protamine and/or the salts thereof can be combined in a wound healing (accelerating) agent for purposes of blood stanching, preservation of their formulation, etc. In such cases, the protamine and the salts thereof are unchanged as the active ingredient for antimicrobial treatment and wound healing acceleration.

In addition, a protamine is a protein material, and, thus, the protamine is a highly safe material without the concern of accumulation in the organs, etc., of in the body, as in the case caused by silver.

Furthermore, quality control for each active ingredient and a process(es) of mixing two or more components, etc., are required in the production of a pharmaceutical composition by combining two or more compounds having different properties and structures as the active ingredients. Such production process become more complicated in comparison with a production process using a single active ingredient. Regarding a protamine and the pharmaceutically acceptable salts thereof, knowledge relating to the safety as a material for a food preservative have been accumulated, and, thus, the quality control methods thereof have been already established.

In a case of production of a wound healing agent by using, as a single active ingredient, at least one compound selected from a protamine and the pharmaceutically acceptable salts thereof, the production process of the wound healing agent can be simplified. The action and the effects are common to the protamine and the pharmaceutically acceptable salts thereof due to the protamine part for the common action and effects. Therefore, the protamine and the pharmaceutically acceptable salts thereof can be deal as equivalent components in their use as the active ingredient of a wound healing agent. Accordingly, also when a wound healing agent is produced by using two or more compounds selected from the protamine and pharmaceutically acceptable salts thereof, they can be deal with as equivalent components in their molecular structures, and their actions and effects, and, thus, the process of producing a wound healing agent can be more simplified.

A wound healing agent according to the present invention comprises at least one compound from a protamine and pharmaceutically acceptable salts thereof, as an active ingredient.

In the wound healing agent of the present invention, antimicrobial agents or wound healing agents other than the protamine and pharmaceutically acceptable salts thereof can be combined within the range so that the advantage of at least one compound from a protamine and pharmaceutically acceptable salts thereof is not impaired.

From the viewpoints of simplification of the production process and the safety of the active ingredient, a preferable wound healing agent comprises an active ingredient, which consists of at least one compound selected from a protamine and pharmaceutically acceptable salts thereof, i. e., the active ingredient is a single active ingredient consisting of at least one compound selected from a protamine and pharmaceutically acceptable salts thereof. A more preferable wound healing agent comprises an active ingredient consisting of one compound selected from a protamine and pharmaceutically acceptable salts thereof.

According to the wound healing agent of the present invention, wound healing accelerating effects can be obtained from both of infection prevention based on the antimicrobial activity and the wound healing accelerating activity provided by at least one compound (hereinafter referred to as "protamine component"), which is selected from a protamine and pharmaceutically acceptable salts thereof.

The recitation "wound" is also designated as "injury" or "external wound or injury", and refers to parts of the body surface, the mucosal surface, or the organ surface, etc., which have tears with opening caused by external stimuli.

The type of the wound to be healed with acceleration by the present invention is not especially limited. Examples of the wounds to be treated include an incision wound, a chop wound, a puncture wound, a contused wound, a laceration wound, an impalement injury, dermabrasion, decollement, an abrasion, a crushed wound, contusion, a firearm wound, a blast injury, a biting wound, excoriation, an explosion injury, a biting wound, a bruise, an external wound, a bedsore, a surgical wound, a burn wound, a gunshot wound, subcutaneous abscess, sutured tear, contaminated tear, stasis ulcer, leg ulcer, foot ulcer, venous ulcer, diabetic ulcer, ischemic ulcer, pressure ulcer, an acute/chronic wound, an open injury and a closed injury.

Subjects in need of a wound treatment to be healed with acceleration by according to the present invention are not especially limited. Exampled of the subjects include animals including human subject. Examples of animal species include dogs, cats, rats, bovines, pigs, sheep, horses and dolphins. Examples of the types of the animals classified with respect to application purpose, pet animals, animals for commercial uses, livestock animals, and animals for competitive uses.

A protamine is a strongly basic protein present in the form of nucleoprotamine bonded to a DNA in a sperm nucleus of fish such as salmon, herring or trout, and is designated as, for example, salmine (salmon) or clupeine (herring) depending on the row material, and there is a slight difference in structure among these. Any of these protamines can be used as the active ingredient.

As the pharmaceutically acceptable salts of a protamine as protamine derivatives, salts with acids or bases can be used. As the acid for obtaining salts, at least one of inorganic acids and organic acids can be used. As the bases for obtaining salts, at least one of inorganic bases and organic bases can be used.

As salts of a protamine, any of pharmaceutically acceptable salts obtained by using the following acids or bases for salt formation can be preferably used.

Examples of inorganic acids and organic acids for forming acid addition salts include hydrochloric acid, nitric acid, phosphoric acid, sulfuric acid, methanesulfonic acid, p-toluenesulfonic acid, dicarboxylic acids such as oxalic acid, malonic acid, succinic acid, maleic acid and fumaric acid, and monocarboxylic acids such as acetic acid, propionic acid and butyric acid.

Examples of inorganic bases for forming salts include hydroxides, carbonates and bicarbonates of ammonia, sodium, lithium, calcium, magnesium, aluminum, etc.

Examples of organic bases for forming salts include mono-, di- and tri-alkylamines such as methylamine, dimethylamine and triethylamine, and mono-, di- and tri-hydroxyalkylamines, guanidine and N-methyl glucosamine.

One of these protamine derivatives or a combination of two or more of these protamine derivatives can be used as needed.

Microorganisms as a target by the antimicrobial effects of a wound healing agent according to the present invention is not especially limited. Examples of the target microorganisms by the antimicrobial effects of the wound healing agent according to the present invention include bacteria, molds and yeasts. The bacteria may include gram-positive bacteria and gram-negative bacteria. Examples of the bacteria include bacteria of the genus Staphylococcus such as Staphylococcus aureus, bacteria of the genus Pseudomonas such as Pseudomonas aeruginosa, Escherichia coli, Bacillus subtilis, bacteria of the genus Salmonella, lactic acid bacteria, etc. Examples of the molds include molds of the genus Candida, etc. Examples of the yeasts include film yeasts, etc.

A wound healing agent of the present invention can be formulated into a dosage form according to an aimed application by using the protamine component as the active ingredient directly, or by appropriately using an excipient, a carrier, a diluent, etc. In order to produce a wound healing agent of the present invention, the protamine component alone can be used as the active ingredient, and furthermore, the protamine component can be used as a single active ingredient.

Examples of a usable dosage form include, a tablet, a capsule, granules, powder, an oral solution, a syrup, an oral jelly, a tablet for oral cavity use, a spray for oral cavity use, a semisolid preparation for oral cavity use, a mouthwash, an injection, an inhalant, an eye drop, an ophthalmic ointment, an ear drop, a nasal drop, a suppository, a semisolid preparation for rectal use, an enema, a vaginal tablet, a suppository for vaginal use, powder for external use, a solid formulation for external use and a solution for external use.

The protamine component in a powder form, etc., can be directly used as a liniment for external use, namely, for topical use. For example, the protamine component can be coated to a substrate, and the coated substrate thus obtained can be applied to a wound site as a wound healing agent. As the substrate, for example, gauze, covering materials of natural sponges, sponges, foam materials, non-woven fabrics, polyurethanes, bandages, adhesive plasters, wound covering or dressing materials such as hydrophilic wound covering or dressing materials and sealing wound covering or dressing materials, dry sheets, dry non-woven fabric sheets, freeze-dried sheets, gel sheets such as solid gel sheets, gel materials such as hydrogel materials and sticky gel materials, etc. Alternatively, a topical liniment, such as an ointment, a lotion, a cream, a rinse or an immersion liquid, containing the protamine component as the active ingredient may be applied to a wound site. The topical liniment may be obtained in a composition or a form that can be applied to a wound site using a spray or an applicator.

A amount of the protamine component contained to be included in a wound healing agent according to the present invention and an application amount thereof to a wound site are not especially limited. They are appropriately selected as effective amounts necessary for treatment, in accordance with the subjects to be treated and the purpose of the treatment. For example, when a protamine is used by applying the protamine to Jintan Medicare sterile pad T (manufactured by Morishita Jintan Co., Ltd.) as a substrate and sticking the pad on a wound site, the amount of the protamine is preferably 0.001% by mass or more than 0.001% by mass, and 50% by mass or less than 50% by mass, with respect to a total amount of the substrate and the protamine. The lower limit value of the amount of the protamine is more preferably 0.01% by mass or more than 0.01% by mass, further preferably 0.1% by mass or more than 0.1% by mass, and most preferably 0.125% by mass or more than 0.125% by mass. The upper limit value of the amount of the protamine is more preferably 10% by mass or less than 10% by mass, further preferably 2% by mass or less than 2% by mass, and most preferably 1.25% by mass or less than 1.25% by mass. An application amount of the protamine in a range of 0.125% by mass to 1.25% by mass is preferably selected, and in this range of the application amount, 1.25% by mass is further preferred. Also, regarding a topical liniment such as an ointment, a lotion, a cream, a rinse or an immersion liquid, etc., the concentration of the protamine component contained in such a liniment can be selected to obtain a similar application amount as described above.

Sterilization treatment may be carried out to the protamine component. The sterilization treatment can be performed to the protamine component in at least one step selected from the steps before supply to the formulation process, during the formulation process and after the formulation process. The sterilization methods to be employed in the sterilization treatment of the protamine is not especially limited as long as the desired effects of the protamine component is not impaired and the desired sterilization effect can be obtained. Examples of the sterilization methods include γ-ray sterilization, radiation sterilization, ethylene oxide gas (EOG) sterilization, gas sterilization, electron beam sterilization, autoclave sterilization, steam sterilization, dry heat sterilization, flame sterilization, boiling sterilization, microwave sterilization, plasma sterilization, ultraviolet sterilization, and filtration by a filter for the use in the form of a solution.

An antibacterial effect and a wound healing accelerating effect can be obtained by the protamine component alone without necessity of the combined use with another active ingredient such as a metal, by using the protamine component having both the antimicrobial activity and the wound healing accelerating effect as the active ingredient for infection prevention based on the antimicrobial activity and the wound acceleration effect. As a result, a wound healing agent may be produced by the protamine component as a single active ingredient. The wound healing agent comprising the protamine component as the single active ingredient enables to intend improvement of the safety by reducing accumulation in the body, toxicity, etc., by a metal such as silver, etc., and to make the production process simpler by allowing the use of a single active gradient, in comparison with the case where different compounds are combined as the active ingredients like a combination of a metal component of silver, etc., and the other component.

The antimicrobial activity is also designated as an antimicrobial action or an antimicrobial effect, and may refer to an effect for inhibiting growth and proliferation of a bacterium or killing a bacterium. In a wound, it is apprehended that a bacterium such as Staphylococcus aureus causes an infectious disease. Since the protamine component has the antimicrobial activity, when the protamine component is applied to a wound site, the infection of the wound site can be prevented.

The antimicrobial activity of the protamine component can be evaluated based on an antimicrobial activity value described in "JIS L 1902:2015 Textiles - Determination of antibacterial activity and efficacy of textile products" (ISO 20743), and when a target material has an antimicrobial activity value of 2.0 or more, it can be determined to have the antimicrobial activity. The protamine component has an antimicrobial activity value against Staphylococcus aureus of 2.0 or more.

The protamine component has, not only the antimicrobial activity, but also the wound healing accelerating effect.

The wound healing accelerating effect refers to an effect for rapidly healing a wound. When a wound is rapidly healed, e.g., a time period when the quality of life is lowered by the wound can be shortened. Since the protamine component has the wound healing accelerating effect, as compared with a case where the wound healing agent is not used, a wound can be more rapidly healed by applying the protamine component. Further, as compared with silver sulfate having the antimicrobial activity, the protamine component can more rapidly heal a wound, and, therefore, the wound healing accelerating effect of the protamine component used according to the present invention results not only from the antimicrobial activity alone, but also from an effect specific to the protamine component.

The wound healing accelerating effect of the protamine component can be evaluated through a healing test using an animal such as a mouse or a rat. Specifically, when healing of a wound is accelerated in a test group using the protamine component as compared with a control group not using the wound healing agent, it can be determined that the protamine component has the wound healing accelerating effect. According to the examination through a healing test performed by the present inventors, it was confirmed that the protamine component can accelerate healing of a wound as compared with a control group in which no wound healing agent was used.

Examples

The present invention will now be concretely described with reference to examples, and it is noted that the present invention is not limited to these examples. [Example 1] Antimicrobial activity Test

A standard cloth was cut into an appropriate size with a mass of 0.40 g $\pm$ 0.05 g. Sterile water (a control sample) and each of the test sample solutions (protamine hydrochloride: manufactured by Maruha Nichiro Corporation, protamine sulfate: manufactured by Maruha Nichiro Corporation, silver sulfate: manufactured by Wako Pure Chemical Industries Ltd.) were prepared and filtered, separately. Each of the filtered solutions was applied to the standard cloth to obtain a solid content of the active ingredient of 0.125% by mass or 1.25% by mass to prepare three types of test pieces, which were then put in separated vials, respectively, which had been autoclaved for sterilization in advance.

A Staphylococcus aureus suspension was inoculated by 0.2 mL each in separate several portions on each test piece. Immediately after the inoculation, 20 mL of a saline for washing out was added to some vials for washing out Staphylococcus aureus cells, and then a viable cell count was measured. The rest of the vials without washing-out was cultured at 37°C $\pm$ 2°C for 18 to 24 hours. After the cultivation, 20 mL of a saline for washing out was added to each vial for washing out Staphylococcus aureus cells, and then a viable cell count was measured. In accordance with the formula (1), an antimicrobial activity value was calculated, and a sample having an antibacterial activity value of 2.0 or more was determined as having the antimicrobial activity.

$$A = (logCt - logC0) - (logTt - logT0) \ldots \text{Formula (1)}$$

A: antibacterial activity value

logCt: arithmetic average common logarithm of viable cell count in a control sample obtained after cultivation for 18 to 24 hours

logC0: arithmetic average common logarithm of viable cell count in a control sample obtained immediately after inoculation

logTt: arithmetic average common logarithm of viable cell count in a test sample obtained after cultivation for 18 to 24 hours

logT0: arithmetic average common logarithm of viable cell count in a test sample obtained immediately after inoculation

The thus obtained results are shown in Table 1. All of protamine hydrochloride, protamine sulfate and silver sulfate had the antimicrobial activity value of 2.0 or more. Thus, it was revealed that all of protamine hydrochloride, protamine sulfate and silver sulfate have the antimicrobial activity.

[Table 1]

Table 1

| Test Sample | Concentration | Antibacterial Activity Value |
|---|---|---|
| Protamine Hydrochlorid | 0.125 % by mass | $\geq$2.0 |
| | 1.25 % by mass | $\geq$2.0 |

(continued)

| Test Sample | Concentration | Antibacterial Activity Value |
|---|---|---|
| Protamine Sulfate | 0.125 % by mass | ≥2.0 |
| | 1.25 % by mass | ≥2.0 |
| Silver Sulfate | 0.125 % by mass | ≥2.0 |
| | 1.25 % by mass | ≥2.0 |

[Example 2] Healing Test

A substrate (Jintan Medicare sterile pad T, manufactured by Morishita Jintan Co., Ltd.) was prepared, and each of filtered test solutions (protamine hydrochloride, protamine sulfate and silver sulfate) was homogeneously applied to each substrate to obtain a solid content of the active ingredient of 1.25% by mass. In a flank region on either side of an eight-week-old BrIHan: WIST@Jcl (GALAS) rat (four rats used in each test group), a full-thickness skin incision wound with a size of 2.5 cm x 2.5 cm was created.

A substrate to which each of the test sample solutions had been applied (test group) or a substrate to which nothing had been applied (control group) was applied, and then fixed with a dressing (3M Tegaderm Smooth Film Roll, manufactured by 3M Japan Limited). The substrate and the dressing were changed once every day, the incision wound was photographed with a digital camera at the time of the change, and then, an area of the wound was obtained by using image analysis software (Image J, National Institutes of Health, USA). Assuming that the area of the wound on the day of creating the wound (day 0 of observation) was 100%, an area ratio (%) of the wound on each observation date was obtained.

The thus obtained results are illustrated in Figure 1. On day 1 to 4 of observation, as compared with a control group and a silver sulfate group of test groups, an effect of accelerating wound healing was observed in a protamine hydrochloride group and a protamine sulfate group of the test groups. Although all of silver sulfate, protamine hydrochloride and protamine sulfate have the antimicrobial activity, the wound healing accelerating effect was found in protamine hydrochloride and protamine sulfate alone, and therefore, it was found that the wound healing accelerating effect of the protamine was not simply derived from the antimicrobial activity but was a peculiar effect of the protamine component obtained separately from the antimicrobial activity.

[Example 3] Examination of Sterilization Treatment

A protamine hydrochloride powder and a protamine sulfate powder were respectively enclosed in sterilization bags (roll bag for IDS, manufactured by Hogy Medical Co., Ltd.). For a control group (not treated), the sterilization treatment was not performed, and the rest was subjected to the EOG sterilization (EOG concentration: 20%, sterilization time: 4 hours, temperature: 55°C) and the $\gamma$-ray sterilization (30 kGy). The standard cloth was cut into an appropriate size with a mass of 0.40 g $\pm$ 0.05 g. Each of the test powders (protamine hydrochloride and protamine sulfate) was dissolved in sterile water, separately, and then the resultant was filtered. The filtered test solutions were individually applied to the standard cloth to obtain a solid content of the active ingredient of 0.125% by mass or 1.25% by mass to prepare test pieces, which were then put in separate vials, which had been autoclaved for sterilization in advance. A Staphylococcus aureus suspension was inoculated by 0.2 mL each in separate several portions on each test piece. Immediately after the inoculation, 20 mL of a saline for washing out was added to some vials for washing out Staphylococcus aureus cells, and then a viable cell count was measured. The rest of the vials without washing-out was cultured at 37°C $\pm$ 2°C for 18 to 24 hours. After the cultivation, 20 mL of a saline for washing out was added to each vial for washing out Staphylococcus aureus cells, and then a viable cell count was measured. In accordance with the formula (1), an antimicrobial activity value was calculated, and a sample having an antimicrobial activity value of 2.0 or more was determined as having the antimicrobial activity.

The thus obtained results are shown in Table 2. Both the protamine hydrochloride and the protamine sulfate having been subjected to the EOG sterilization and the $\gamma$-ray sterilization have an antimicrobial activity value of 2.0 or more, and thus, it was confirmed that the antibacterial properties of protamine hydrochloride and protamine sulfate are not lost through the sterilization treatment.

[Table 2]

Table 2

| Test Sample | Concentration | Treatment | Antibacterial Activity Value |
|---|---|---|---|
| Protamine Hydrochloride | 1.25 % by mass | Not Treated | ≥2.0 |
| | | EOG Sterilization | ≥2.0 |
| | | γ-ray Sterilization | ≥2.0 |
| Protamine Sulfate | 1.25 % by mass | Not Treated | ≥2.0 |
| | | EOG Sterilization | ≥2.0 |
| | | γ-ray Sterilization | ≥2.0 |

Industrial Applicability

According to the present invention, both of an antimicrobial effect and a wound healing accelerating effect can be obtained also by the protamine component alone and, thus, a wound healing agent comprising the protamine component as a single active ingredient can be provided.

**Claims**

1.   A wound healing agent comprising at least one compound selected from a protamine and pharmaceutically acceptable salts thereof as an active ingredient for antimicrobial activity and wound healing acceleration.

2.   The wound healing agent according to claim 1, wherein the active ingredient is a single active ingredient consisting of at least one compound selected from a protamine and pharmaceutically acceptable salts thereof.

3.   The wound healing agent according to claim 1, comprising one compound selected from a protamine and pharmaceutically acceptable salts thereof as a single active ingredient.

4.   A method of using, at least one compound selected from a protamine and pharmaceutically acceptable salts thereof in production of a wound healing agent comprising an active ingredient for antimicrobial activity and wound healing acceleration, as the active ingredient.

5.   The method according to claim 4, wherein the active ingredient is a single active ingredient consisting of at least one component selected from a protamine and pharmaceutically acceptable salts thereof.

6.   The method according to claim 4, wherein one compound selected from a protamine and pharmaceutically acceptable salts thereof is used as a single active ingredient of the wound healing agent.

7.   Use of at least one compound selected from a protamine and pharmaceutically acceptable salts thereof for antibacterial activity and wound healing acceleration.

8.   The use according to claim 7, wherein the active ingredient is a single active ingredient consisting of at least one selected from a protamine and pharmaceutically acceptable salts thereof.

9.   The use according to claim 7, wherein one compound selected from a protamine and pharmaceutically acceptable salts thereof is used as a single active ingredient of the wound healing agent.

10.   A treatment method for obtaining antibacterial activity and wound healing acceleration of a wound of a patient, comprising applying a wound healing agent containing at least one compound selected from a protamine and pharmaceutically acceptable salts thereof as an active ingredient for wound healing, to the wound.

11.   The treatment method according to claim 10, wherein the active ingredient is a single active ingredient consisting of at least one compound selected from a protamine and pharmaceutically acceptable salts thereof.

12. The treatment method according to claim 10, wherein one compound selected from a protamine and pharmaceutically acceptable salts thereof is used as a single active ingredient of the wound healing agent.

Figure 1

## INTERNATIONAL SEARCH REPORT

| International application No. |
|---|
| PCT/JP2017/036060 |

A.  CLASSIFICATION OF SUBJECT MATTER
*A61P17/02*(2006.01)i, *A61K35/60*(2006.01)i, *A61K38/00*(2006.01)i, *A61P31/04*
(2006.01)i

According to International Patent Classification (IPC) or to both national classification and IPC

B.  FIELDS SEARCHED

Minimum documentation searched (classification system followed by classification symbols)
A61P17/02, A61K35/60, A61K38/00, A61P31/04

Documentation searched other than minimum documentation to the extent that such documents are included in the fields searched
| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/MEDLINE/EMBASE/BIOSIS(STN)

C.  DOCUMENTS CONSIDERED TO BE RELEVANT

| Category* | Citation of document, with indication, where appropriate, of the relevant passages | Relevant to claim No. |
|---|---|---|
| X | Masataka KIMURA et al., "Immunohistochemical Study of Protamine-reduced Peptides on Wound Healing in an Experimental Rat Skin Wound Healing Model", Jpn Pharmacol Ther, 2014, vol.42, no.3, pages 203 to 210, particularly, Abstract, page 204, left column, 1st paragraph | 1-12 |
| X | WO 2010/110067 A1  (Maruha Nichiro Foods, Inc.), 30 September 2010 (30.09.2010), paragraphs [0044], [0045]; example 9 & US 2012/0021063 A1 paragraphs [0121], [0122]; example 9 & EP 2412795 A1        & KR 10-2011-0139300 A & TW 201036653 A | 1-12 |

☒  Further documents are listed in the continuation of Box C.      ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 24 October 2017 (24.10.17) | 07 November 2017 (07.11.17) |

| Name and mailing address of the ISA/ | Authorized officer |
|---|---|
| Japan Patent Office | |
| 3-4-3,Kasumigaseki,Chiyoda-ku, | |
| Tokyo 100-8915,Japan | Telephone No. |

Form PCT/ISA/210 (second sheet) (January 2015)

**INTERNATIONAL SEARCH REPORT**

| International application No. |
| --- |
| PCT/JP2017/036060 |

C (Continuation).   DOCUMENTS CONSIDERED TO BE RELEVANT

| Category* | Citation of document, with indication, where appropriate, of the relevant passages | Relevant to claim No. |
| --- | --- | --- |
| X | REIMONDEZ-TROITINO, Sonia, et al., Polymeric nanocapsules: a potential new therapy for corneal wound healing, Drug Deliv and Transl Res, 2016.07.08, Vol.6, p708-721, Abstract, Figs 5,6 | 1-12 |
| X | Yuki TAKABAYASHI et al., "Bunso Saihiso Rat deno Fragmin·Protamine Biryushi Gan'yu PRP no Sosho Chiyu Sokushin Koka no Kensho to sono Rinsho Oyo ni Mukete", Skin Surg, 2015, vol.24, no.1, page 66, Title P8 | 1,4,7,10 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016040294 A
- JP 2012515726 A
- JP S62501628 A
- JP 2002502413 A

**Non-patent literature cited in the description**

- **ATIYEH BS ; COSTAGLIOLA M ; HAYEK SN ; DI-BO SA.** Effect of silver on burn wound infection control and healing: Review of the literature. *Burns,* 2007, vol. 33, 139-148